Europäisches Patentamt

European Patent Office

Office européen des brevets

(11) Numéro de publication: **0 375 525**
**A1**

# DEMANDE DE BREVET EUROPEEN

(21) Numéro de dépôt: 89403509.6

(22) Date de dépôt: **15.12.89**

(51) Int. Cl.5: **C12N 15/52, C12P 17/18,**
**C12N 1/20, //C12N1:20**

(30) Priorité: **16.12.88 FR 8816639**

(43) Date de publication de la demande:
**27.06.90 Bulletin 90/26**

(84) Etats contractants désignés:
**CH DE LI**

(71) Demandeur: **TRANSGENE S.A.**
**16, rue Henri Régnault**
**F-92400 Courbevoie(FR)**

(72) Inventeur: **Gloeckler, Rémi**
**10, quai Chanoine Winterer**
**F-67000 Strasbourg(FR)**
Inventeur: **Speck, Denis**
**4, rue du Bitzen**
**F-67200 Eckbolsheim(FR)**
Inventeur: **Sabatie, Jean**
**100, rue de la Ziegelau**
**F-67100 Strasbourg(FR)**
Inventeur: **Brown, Steve**
**49, rue des Mésanges**
**F-67200 Obershaeffolsheim(FR)**
Inventeur: **Lemoine, Yves**
**4, rue des Alisiers**
**F-67100 Strasbourg(FR)**

(74) Mandataire: **Warcoin, Jacques et al**
**Cabinet Régimbeau 26, avenue Kléber**
**F-75116 Paris(FR)**

(54) **Nouvelle souche d'escherichia coli recombinante et procédé pour la preparation de biotine utilisant ladite souche.**

(57) La présente invention concerne une souche de E. coli recombinante, caractérisée en ce qu'elle comporte au moins certains des gènes bio XWF et bio DAYB d'une souche de B. sphaericus.

EP 0 375 525 A1

# NOUVELLE SOUCHE D'ESCHERICHIA COLI RECOMBINANTE ET PROCEDE POUR LA PREPARATION DE BIOTINE UTILISANT LADITE SOUCHE

La présente invention concerne la préparation de biotine par fermentation en utilisant les techniques de l'ADN recombinant.

La biotine est synthétisée naturellement par certains microorganismes, en particulier des bactéries du genre Bacillus telles que Bacillus sphaericus, ou encore des bactéries telles que Escherichia coli.

L'étude systématique de la production de la biotine à partir de l'acide pimélique au cours de fermentations industrielles a montré que certains microorganismes, particulièrement ceux appartenant au genre B. sphaericus, hyperproduisent des intermédiaires de la biotine (ci-après appelés vitamères) de même que la biotine. En particulier, il est connu que l'addition de pimélate au milieu de culture permet d'augmenter de manière sensible la biosynthèse de la biotine et de ses différents vitamères (1). Cette propriété a été étudiée en détail chez B. sphaericus où les différents enzymes impliqués dans la biosynthèse ont été clairement identifiés (2).

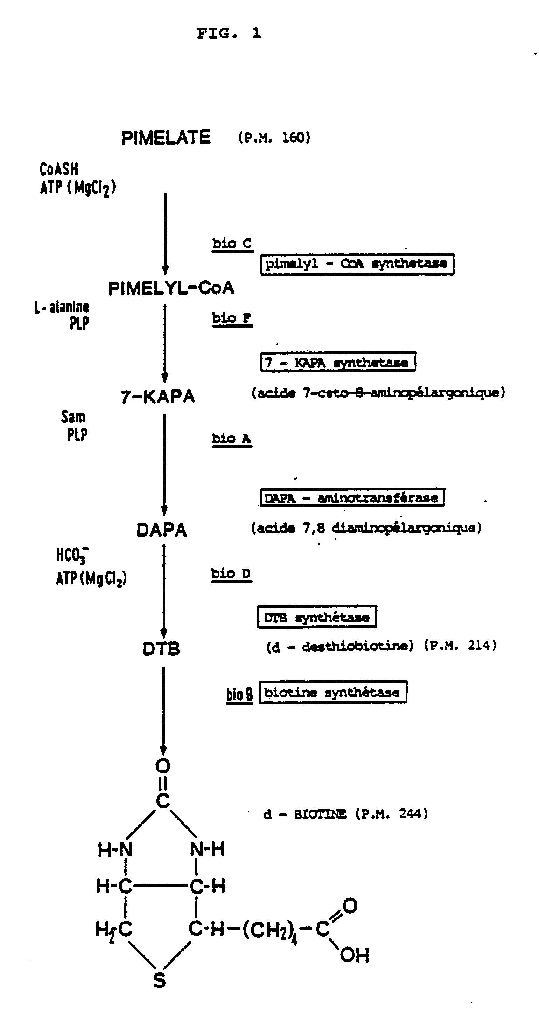

La figure 1 schématise la chaîne de biosynthèse de la biotine à partir de l'acide pimélique dans ce type de microorganisme. Cette chaîne de biosynthèse comprend 5 étapes enzymatiques différentes dans lesquelles les gènes mis en oeuvre sont dénommés successivement bioC, bioF, bioA, bioD et bioB.

L'addition de pimélate au milieu de culture induit une augmentation d'un facteur 1 000 du niveau basal de vitamères et de biotine.

Ces constatations observées chez B. sphaericus n'ont pu être confirmées chez une autre espèce de microorganisme producteur naturel de biotine, la bactérie E. coli (3).

De nombreux travaux ont porté sur l'étude de la voie de biosynthèse de la biotine chez les microorganismes appartenant à cette espèce, une meilleure connaissance de cette voie de biosynthèse devant permettre d'envisager l'utilisation des techniques du génie génétique pour améliorer le rendement de synthèse en biotine.

Cinq gènes de structures, liés en un opéron ont été identifiés. Le rôle des 4 premiers (A, B, F, D) dans la transformation du piméloyl CoA en biotine a été démontré. Le cinquième gène, bioC, et le gène bioH, non lié génétiquement aux 5 gènes précédents, font encore l'objet d'études visant à démontrer leur implication commune ou individuelle dans la biosynthèse du piméloyl CoA, à partir d'un précurseur restant à préciser.

Plusieurs publications de brevet décrivent l'utilisation des techniques du génie génétique pour augmenter la production de biotine par la bactérie E. coli.

En particulier, la publication internationale de brevet WO 87/01391 décrit le clonage et l'hyperexpression de l'opéron biotine de E. coli dans E. coli. Cette publication décrit une augmentation sensible du niveau de biotine produite. Toutefois, la biotine est obtenue en utilisant notamment le glucose comme précurseur, mais jamais le pimélate.

Or, pour envisager la production de biotine de façon réellement économique sur le plan industriel, à partir de bactéries E. coli recombinantes, il est important de pouvoir utiliser le pimélate comme produit de départ.

La présente invention vise à fournir un nouveau procédé de préparation de biotine par fermentation de souches de E. coli recombinantes, qui permette d'obtenir de la biotine à partir du pimélate introduit dans le milieu de culture comme précurseur de la biosynthèse.

C'est pourquoi, l'invention a d'abord pour objet des souches de E. coli recombinantes qui contiennent au moins certains des gènes bioXWF et bioDAYB d'une souche de B. sphaericus, de préférence la totalité de ces gènes.

Ces gènes peuvent être portés par un ou plusieurs vecteurs plasmidique ou bien, éventuellement, être en partie intégrés dans le génome bactérien. Il est également possible de prévoir que certains de ces gènes sont les gènes de E. coli.

Les plasmides d'expression dans E. coli ou le plasmide d'intégration dans E. coli sont connus. Dans le cas présent, on préfère utiliser un seul plasmide portant l'ensemble des gènes de B. sphaericus en conservant l'organisation des deux blocs et, de façon encore préférée, les deux blocs seront en orientation opposée.

Les promoteurs préférés sont les promoteurs inductibles. Par exemple, le promoteur de l'opéron tryptophane de E. coli ou l'opéron lactose de E. coli.

La Demanderesse a déjà décrit dans la publication européenne de brevet 0 266 240 le clonage et la caractérisation des gènes correspondant aux différentes étapes de la voie de biosynthèse de la biotine dans une bactérie du genre B. sphaericus, en particulier la bactérie B. sphaericus IFO 3525. Ces gènes ont été définis comme correspondant aux LORF DAYB, XWF et il a été constaté qu'ils étaient organisés sous forme de deux unités où les gènes DAYB sont liés et respectivement XWF sont liés.

L'organisation moléculaire de ces deux groupes de gènes est très similaire et il convient de noter que l'on peut identifier, en amont des gènes bioD et bioX, en tête de chaque unité, une structure commune et identique de 15 paires de bases intégrée dans une structure palindromique typique des régions régulatrices, très similaire dans les deux cas. Les protéines correspondant aux gènes bioX et bioY, dont la fonction catalytique dans la transformation du pimélate en biotine n'a pas été encore clairement définie, présentent toutes les caractéristiques des protéines hydrophobes impliquées dans les membranes.

La présente invention concerne, en outre, un procédé de préparation de biotine par fermentation de souches de E. coli recombinantes, caractérisé en ce qu'on fait fermenter un milieu de culture contenant au moins de l'acide pimélique ou l'un de ses dérivés par les souches de E. coli selon l'invention et en ce qu'on récupère la biotine formée.

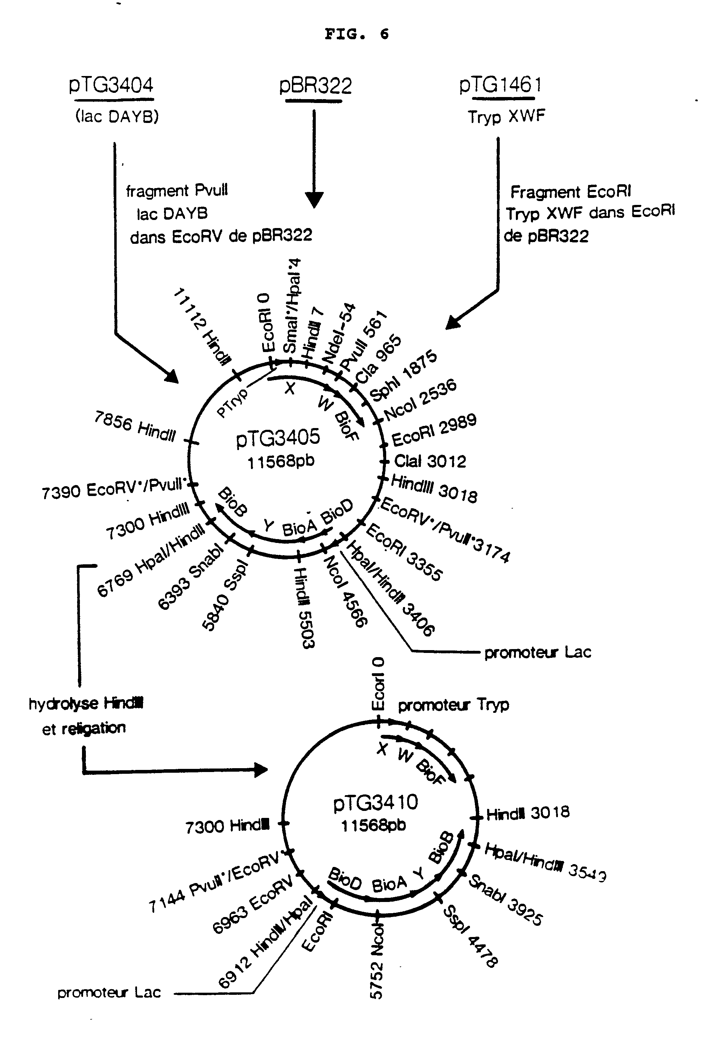

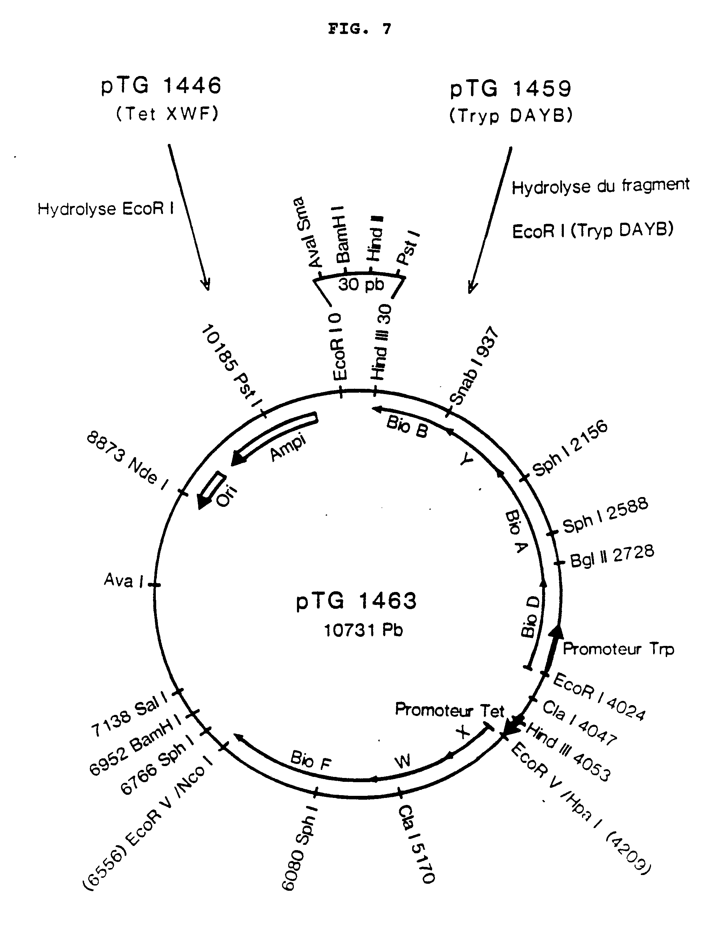

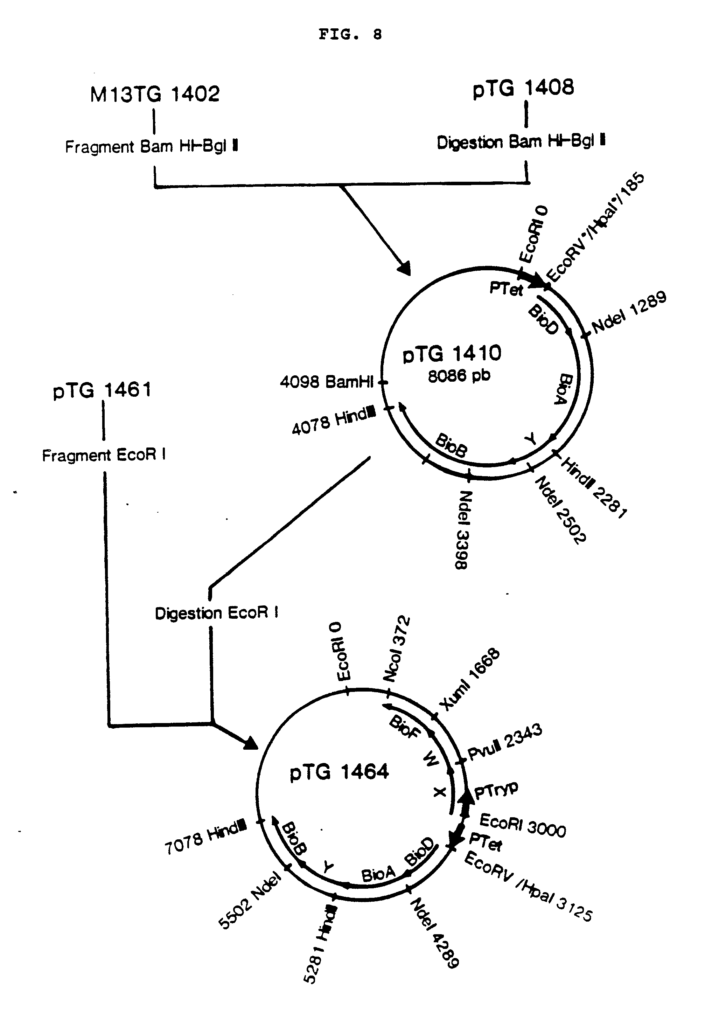

D'autres caractéristiques et avantages de l'invention apparaîtront au cours de la description détaillée suivante illustrée par les figures 1 à 8.
- La figure I rappelle de façon schématique la chaîne de biosynthèse de la biotine.
- La figure 2 schématise les plasmides pTG1461 et pTG3400.
- La figure 3 schématise le plasmide pTG3401.
- La figure 4 schématise le plasmide pTG1459.
- La figure 5 schématise le plasmide pTG3404.
- La figure 6 schématise le plasmide pTG3405.
- La figure 7 schématise le plasmide pTG1463.
- La figure 8 schématise le plasmide pTG1464.

Tous les plasmides décrits dans la suite de la description comprennent, outre le fragment approprié de la souche B. sphaericus IFO 3525, les éléments suivants :
- Un gène permettant d'assurer dans des conditions de culture définies l'expression des gènes.
- Des séquences propres à assurer la régulation de l'expression de ces gènes.
- Une origine de réplication dans E. coli.

La publication de brevet européen 0 266 240 citée décrit le clonage du fragment d'ADN HpaI-sspI de la souche B. sphaericus IFO 3525 (coordonnées 1107 à 4011 de la séquence publiée dans cette publication comprenant les ORF XWF et la séquence correspondant à un terminateur de transcription).

Dans une première série d'expériences, on décrit la transformation in vivo du précurseur pimélate en Kapa, par l'expression dans des conditions de culture définies, des gènes bioXWF. Les trois plasmides exemplifiés diffèrent essentiellement par la nature du promoteur assurant l'expression des gènes.

Exemple 1 : Transformation de pimélate en Kapa

La construction du plasmide pTG1446 a été décrite dans la publication de brevet déjà citée et dont le contenu est ici incorporé par référence. Les gènes XWF sont sous le contrôle du promoteur constitutif du gène de résistance à la tétracycline.

Dans le plasmide pTG1461, les gènes XWF sont sous le contrôle du promoteur inductible de l'opéron tryptophane de E. coli.

Dans le plasmide pTG3401, ils sont sous le contrôle du promoteur inductible de l'opéron lactose de E. coli.

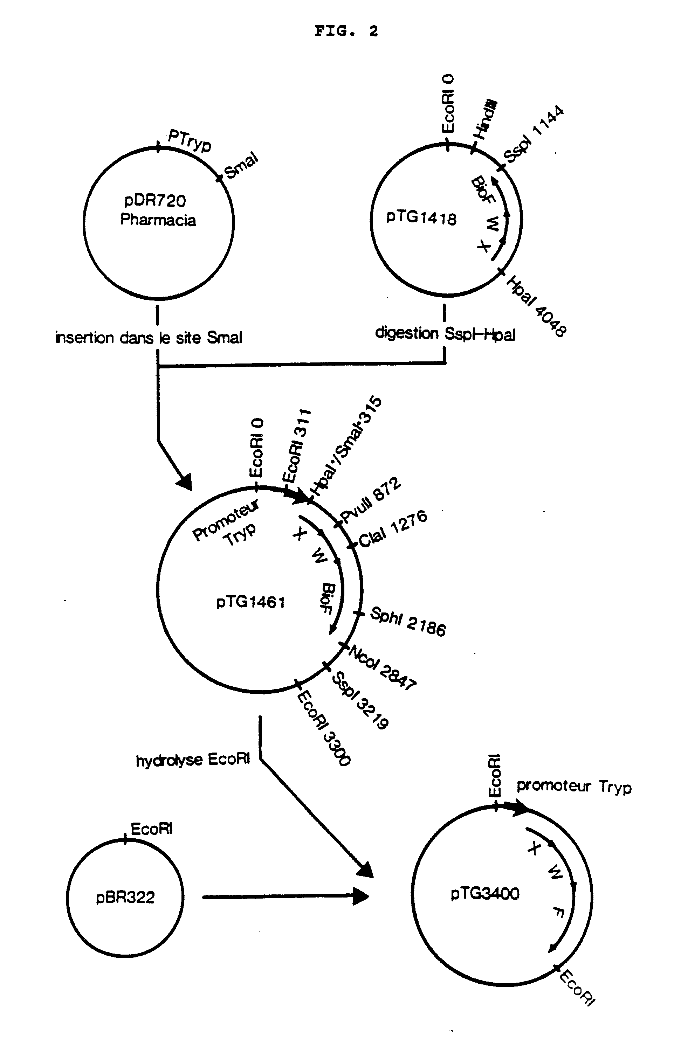

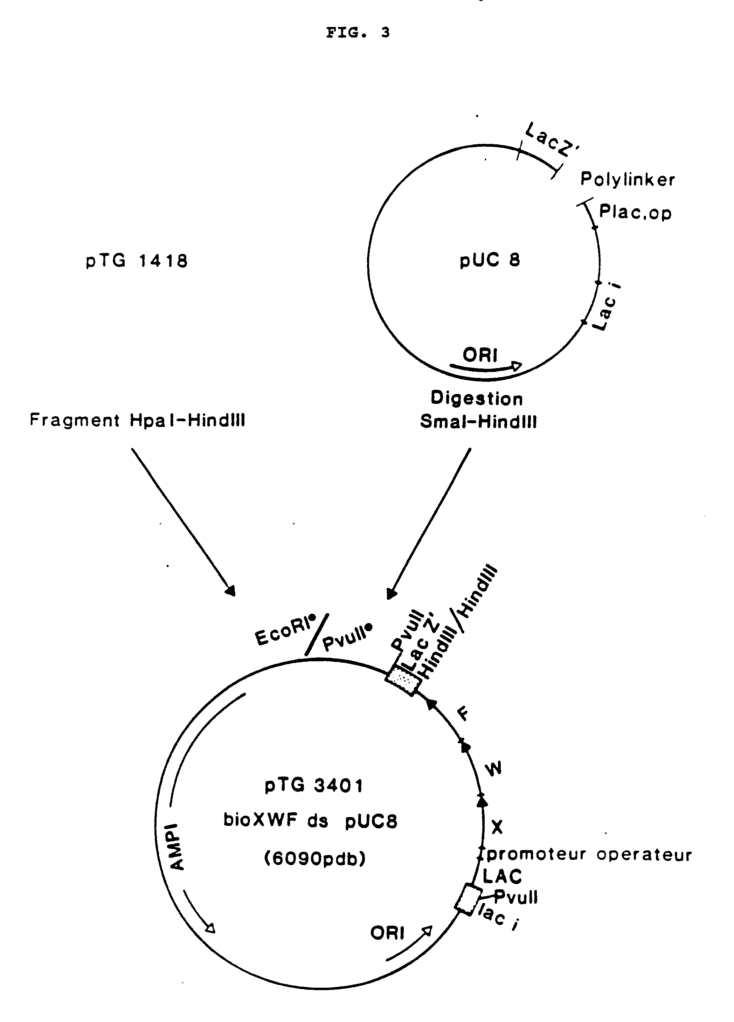

Ces trois constructions sont représentées aux figures 2 et 3.

Ces plasmides sont introduits dans la souche E. coli MC169 (F⁻, leu 6, pur E42, trp E38, thi-1, ara-14, lacY1, galK2, xyl-5, str-109, cap R9, gal 4, sup E44, λ⁻) (4). La souche recombinante est mise en culture à 37°C pendant 48 heures dans du milieu PC 1.2 (milieu PC pour 1 litre : glycérol : 20 g; protéose peptone : 30 g; "casamino acids vitamin frée" : 20 g; $K_2HPO_4$ : 1 g; Kcl : 0,5 g; $MgSO_4 - 7 H_2O$ : 0,5 g; $FeSO_4 - 7 H_2O$ : 0,01 g; $MnSO_4 - 6 H_2O$ : 0,01 g - le milieu PC 1.2 est obtenu en multipliant tous les constituants par 1.2).

A ce milieu sont ajoutés 100 g/ml d'ampicilline et 300 μg/ml de pimélate neutralisé à pH 7. La culture est ensuite diluée au 1/100e dans ml de même milieu, et agitée jusqu'à atteindre une absorbance à 660 nm voisine de 0.2. On ajoute alors l'inducteur (IAA 20 ug/ml dans le cas du promoteur TRYP, IPTG 1 mM dans le cas du promoteur lac) et la culture est poursuivie à 37°C.

Une aliquote du surnageant (5 μl) est ensuite mise en chromatographie sur plaque de silice (solvant chromato = n butanol : 60 (vol/vol)/acide acétique : 15 (vol/vol)/eau : 25 (vol/vol), migration du solvant : 10 cm) pour séparer les vitamères et la biotine produits.

3

Après migration spécifique à chaque vitamère, le Kapa est évalué quantitativement par dosage biologique en utilisant une souche de S. cerevisiae de collection : ATCC 7754.

Après 24 heures de culture, les niveaux de production de Kapa sont indiqués dans le tableau suivant

| Souche/plasmide | Quantité de Kapa ($\mu$g/ml de surnageant en équivalent de biotine) | |
|---|---|---|
| | avec pimélate | sans pimélate |
| E.coli MC169/pTG 1446 | 10 | <0.1 |
| E.coli MC169/pTG 1461 | 120 | <0.1 |
| E.coli MC169/pTG 3401 | 170 | <0.1 |

Ces résultats mettent clairement en évidence deux points : d'une part la synthèse de Kapa ne s'effectue pas en l'absence de pimélate, et le flux de Kapa est fortement augmenté lorsque le pimélate est mis comme précurseur dans le milieu de culture ; d'autre part, pour une quantité définie de précurseur pimélate, la quantité de Kapa produite est influencée par la force du promoteur utilisé, un promoteur réputé fort permettant d'obtenir effectivement davantage de Kapa. On utilisera donc de préférence un promoteur inductible tel que le promoteur lac.

### Exemple 2 : Transformation de DTB en biotine

Cette deuxième série d'expériences permet de vérifier la fonctionnalité des gènes DAYB de B. sphaericus IFO 3525 dans E. coli, par la biotransformation in vivo du vitamère DTB, ajouté comme précurseur à la culture, en biotine.

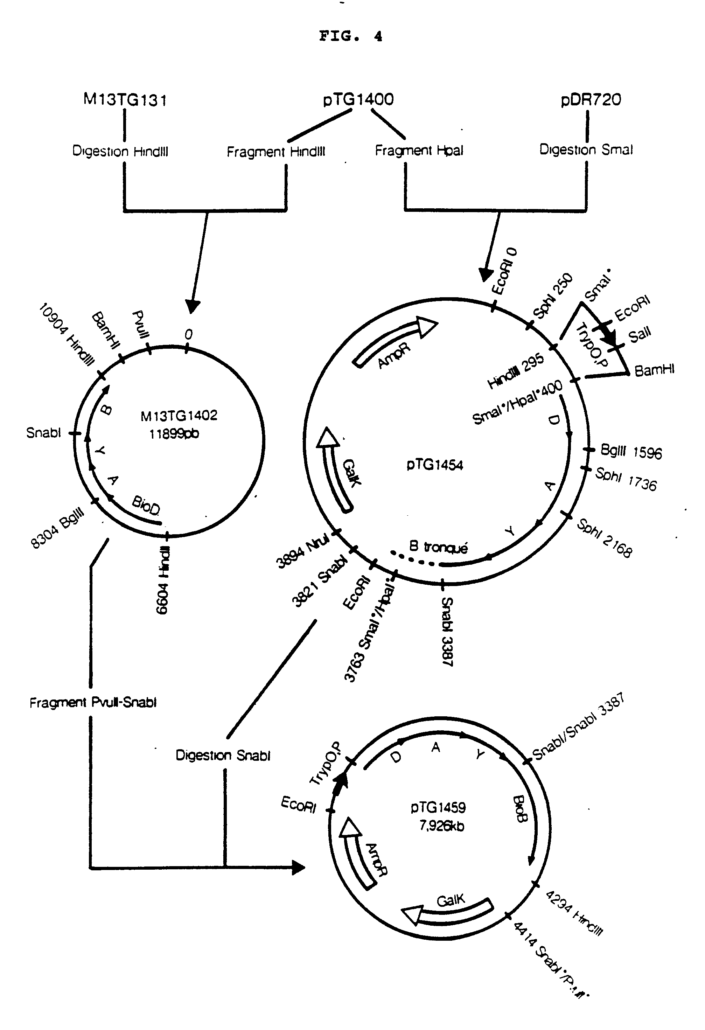

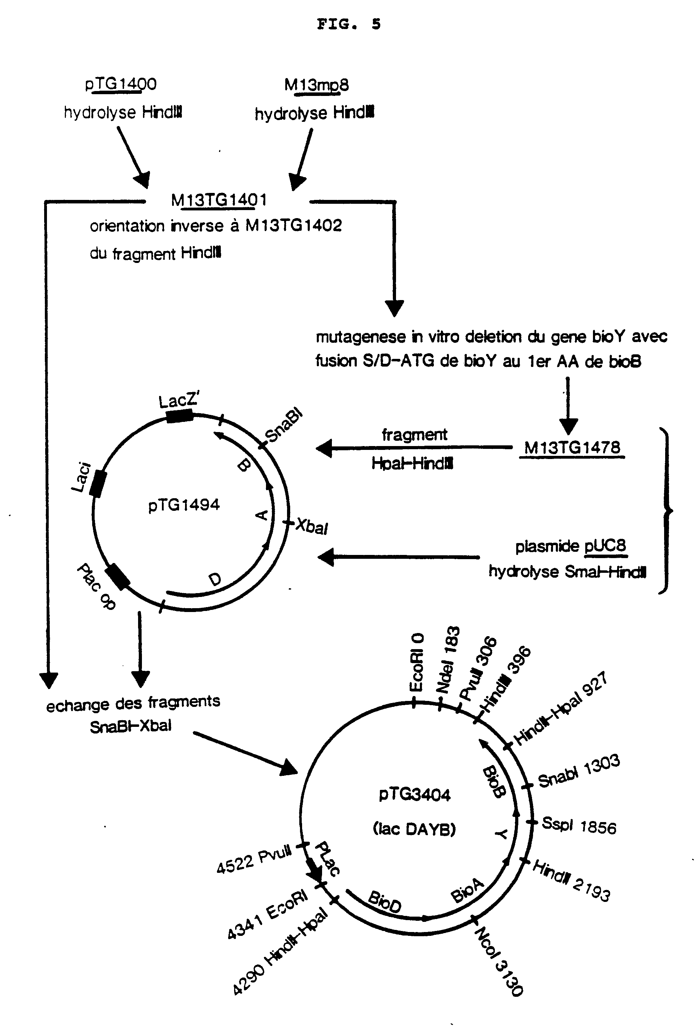

Les plasmides pTG1459 et pTG3404 représentés aux figures 4 et 5 comprennent tous deux des clones du fragment d'ADN HpaI-HindIII de la souche B. sphaericus IFO 3525 (coordonnées 483-4377 de la séquence publiée dans la demande de brevet européen 0 266 240 déja citée, comprenant les ORF DAYB et la séquence correspondant à un terminateur de transcription).

Dans le plasmide pTG1459, les gènes DAYB sont sous le contrôle du promoteur inductible de l'opéron tryptophane de E. coli (Tryp.).

Dans le plasmide pTG3404, ils sont sous le contrôle du promoteur inductible de l'opéron lactose de E. coli (lac).

Comme dans l'exemple 1, ces plasmides sont introduits dans la souche E. coli MC169 et, à la place du pimélate, on ajoute au milieu de culture le DTB comme précurseur, à raison de 100 ug/ml. La biotine produite est dosée biologiquement par essai microbiologique avec Lactobacillus Plantarum ATCC 8014.

Après 24 heures de culture, les niveaux de production de biotine sont indiqués dans le tableau suivant :

| Souche/plasmide | Quantité de biotine ($\mu$g/ml de surnageant) | |
|---|---|---|
| | avec DTB | sans DTB |
| E.coli MC169/pTG 1459 | 0.5 | <0.1 |
| E.coli MC169/pTG 3404 | 15 | <0.1 |

Comme dans l'exemple 1, ces résultats mettent en évidence que, pour une quantité définie de précurseur, la quantité de vitamère produite est influencée par la force du promoteur utilisé.

### Exemple 3 : Transformation de pimélate en biotine

Dans cette troisième série d'expériences, les souches E. coli sont transformées avec des vecteurs permettant l'expression des deux groupes de gènes XWF et DAYB.

Différentes constructions ont été réalisées :

- Dans le plasmide pTG3405, les gènes XWF sont sous le contrôle du promoteur tryp, et les gènes DAYB sont sous le contrôle du promoteur lac, et les deux blocs d'expression sont dans la même orientation.

- Le plasmide pTG3410 ne diffère du plasmide pTG3405 que par le fait que les deux blocs d'expression sont orientés en sens inverse.

- Dans le plasmide pTG1463, les gènes XWF sont sous le contrôle du promoteur constitutif du gène de résistance à la tétracycline et les gènes DAYB sont sous le contrôle du promoteur tryp.

- Dans le plasmide pTG1464, les gènes XWF sont sous le contrôle du promoteur tryp et les gènes DAYB sont sous le contrôle du promoteur constitutif du gène de résistance à la tétracycline de E. coli.

Ces différentes constructions ont été introduites d'une part dans la souche E. coli MC 169 déjà citée et d'autre part dans la souche E. coli C268 (str-r, his⁻, bioAΔ224) (5).

Les conditions de culture sont identiques à celles décrites en relation avec l'exemple 1. La biotine produite est dosée comme indiqué à l'exemple 2.

Les niveaux de production de biotine à partir de pimélate au bout de 24 heures de culture sont indiqués dans le tableau suivant

| Souche/plasmide | Quantité de biotine (μg/ml de surnageant) | |
|---|---|---|
| | avec pimélate | sans pimélate |
| E.coli MC169/pTG 1463 | 0.26 | <0.1 |
| E.coli MC169/pTG 1464 | 0.58 | <0.1 |
| E.coli MC169/pTG 3405 | 0.82 | <0.1 |
| E.coli MC169/pTG 3410 | 1.05 | <0.1 |
| E.coli C268/pTG 1463 | 0.58 | <0.1 |
| E.coli C268/pTG 1464 | 1.2 | <0.1 |
| E.coli C268/pTG 3405 | 0.9 | <0.1 |
| E.coli C268/pTG 3410 | 1.9 | <0.1 |

A partir de ce tableau, on peut faire les constatations suivantes :

- Il est clair que le pimélate peut être utilisé comme précurseur pour la production de biotine par des bactéries E. coli.

- On observe une augmentation sensible de la quantité de biotine produite, tous les autres paramètres étant inchangés, lorsqu'on change le sens de transcription des blocs d'expression des deux groupes de gènes (comparaison entre pTG3405 et pTG3410). C'est pourquoi, suivant une caractéristique avantageuse de l'invention, on utilisera des plasmides dans lesquels les deux blocs d'expression sont orientés en sens inverse.

- La souche E. coli 268 paraît plus performante. C'est pourquoi, on a choisi cette souche pour la mise en fermentation.

Exemple 4 : Culture en fermenteur de la souche E. coli C268/pTG3405

Une préculture à 30° C en erlenmeyer de 2 litres est lancée à partir de plusieurs colonies de cellules transformées obtenues sur milieu sélectif. Le milieu de préculture (100 ml de PC 1.2 additionné de pimélate 0,3 mg/ml) est inoculé à une absorbance de 0,1 et on observe après 15 heures une DO de l'ordre de 10, ce qui signifie que les souches ont correctement poussé.

Un volume adéquat de cette culture est utilisé pour inoculer à une absorbance de 0,1, un litre de milieu PC 1.2 additionné de pimélate 0,3 mg/ml contenu dans un fermenteur du type Biolafitte de volume 2 litres.

La fermentation se poursuit dans les conditions suivantes : température 37° C, pH 7 maintenu par addition soit de soude soit d'acide sulfurique concentré. La quantité d'oxygène dissous est suivie jusqu'à ce que la pression partielle en oxygène atteigne 30 %, cette valeur est ensuite maintenue par contrôle de l'agitation et addition d'oxygène pur.

L'induction du promoteur TRP se fait par ajout de 20 μg/ml d'IAA à une absorbance de 0,3. La consommation du glycénol du milieu PC 1.2 est suivie par dosage au moyen d'un kit de dosage commercialisé par la société Boehringer et sa concentration dans le milieu de culture est maintenue entre 0 et 10 g/l par addition discontinue de solution de glycérol concentrée à 60 poids/poids.

Les concentrations en biotine et vitamères après 30 heures de culture sont rassemblées dans le tableau suivant :

| Essai | DO maximale | Biotine mg/ 1 | en équivalent biotine | | |
|---|---|---|---|---|---|
| | | | Kapa (mg/1) | DTB (mg/1) | Vitamères totaux (mg/1) |
| 1 | 56 | 15 | 35 | - | 144 |
| 2 | 70 | 16 | 31 | 40 | 160 |

Le dosage des vitamères totaux comprend les vitamères suivants : Kapa, Dapa, DTB, biotine. Il se fait par essai microbiologique avec la souche 5. cerevisiae ATCC 7754.

La souche Escherichia coli C268/pTG3405 a été déposée à la Collection Nationale de Cultures de Microorganismes de l'Institut Pasteur le 9 décembre 1988 sous le n° I-830.

BIBLIOGRAPHIE

1. IZUMI Y., TANI Y. and YAMADA H.; Biotin biosynthesis in microorganisms, Bull. Inst. Chem. Res., Kioto University. 58 (1980) 434-447.

2. IZUMI Y., KANO Y., INAGAKI K., KAWASE N., TANI Y. and YAMADA H.; Characterization of biotin biosynthetic enzymes of B. sphaericus : a dethiobiotin producing bacterium, Agric. Biol. Chem. 45 (1981) 1983-1989.

3. PAI C.H. and LICHSTEIN H.C., The biosynthesis of biotin in microorganisms; 1 : The physiology of biotin synthesis in E. coli, Biochem. Biophys. Acta 100 (1965) 28-35.

4. ZEHNBAUER et al.; Journal of bacteriology, August 1980 852-863.

5. CLEARY et al.; Journal of bacteriology, November 1972, 830-839.

## Revendications

1) Souche de E. coli recombinante, caractérisée en ce qu'elle comporte au moins certains des gènes bio XWF et bio DAYB d'une souche de B. sphaericus.

2) Souche de E. coli selon la revendication I, caractérisée en ce que lesdits gènes proviennent de la souche B. sphaericus IFO 3525.

3) Souche de E. coli selon l'une des revendications 1 ou 2, caractérisée en ce que tous les gènes sont portés par un seul vecteur plasmidique.

4) Souche de E. coli selon la revendication 3, caractérisée en ce que dans le vecteur plasmidique l'expression des gènes bio XWF d'une part et bio DAYB d'autre part est dirigée par deux blocs d'expression différents.

5) Souche de E. coli selon la revendication 3, caractérisée en ce que les deux blocs d'expression comportent des promoteurs inductibles.

6) Souche de E. coli selon l'une des revendications 4 ou 5, caractérisée en ce que les deux blocs d'expression sont orientés en sens inverse.

7) Procédé de préparation de biotine par fermentation de souches de E. coli recombinantes, caractérisé en ce qu'on fait fermenter un milieu de culture contenant au moins de l'acide pimélique ou l'un de ses dérivés par les souches de E. coli selon l'une des revendications 1 à 6 et en ce qu'on récupère la biotine formée.

FIG. 1

FIG. 2

FIG. 3

FIG. 4

**FIG. 5**

## FIG. 6

FIG. 7

FIG. 8

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,X | EP-A-0 266 240 (TRANSGENE S.A.)<br>* Page 3, ligne 63 - page 4, ligne 35; exemple 7; revendications 24,25,27,29 *<br>--- | 1,2,7 | C 12 N 15/52<br>C 12 P 17/18<br>C 12 N 1/20 //<br>(C 12 N 1/20<br>C 12 R 1:19 ) |
| A | EP-A-0 240 105 (NIPPON ZEON LTD)<br>----- | | |

DOMAINES TECHNIQUES RECHERCHES (Int. Cl.5)

C 12 N
C 12 P

**Le présent rapport a été établi pour toutes les revendications**

| Lieu de la recherche | Date d'achèvement de la recherche | Examinateur |
|---|---|---|
| LA HAYE | 07-03-1990 | DESCAMPS J.A. |

EPO FORM 1503 03.82 (P0402)